# EUROPEAN PATENT APPLICATION

(11) **EP 4 230 638 A1**
(43) Date of publication of application: **23.08.2023**
(21) Application number: 22382122.4
(22) Date of filing: 16.02.2022
(51) Int. Cl.: C07K 7/06, C07K 14/46, A61K 8/64, A61Q 19/08

(54) **PEPTIDES AND COMPOSITIONS FOR USE IN COSMETICS**

(71) Applicant: Lipotrue, S.L., 08850 Gava (ES)
(72) Inventor: Grau-Campistany, Ariadna, 08028 Barcelona (ES); Pastor, Silvia, 03540 Alicante (ES); Carulla, Patricia, 08019 Barcelona (ES); Escudero, Juan Carlo, 08021 Barcelona (ES); Klein, Marco Jan, 08902 L'Hospitalet de Llobregat (ES); Trim, Steven Anthony, 08850 GAVA (ES)
(74) Representative: Clarke, Modet y Cía., S.L.

(57) **Abstract**

The present invention refers to peptides of between 4 and 8 amino acids capable of decreasing the activity and/or signal of at least one type of sodium channel. Said peptides are also capable of promoting the production of extracellular matrix. Also disclosed herein are compositions comprising said peptides and uses of the peptides and the compositions of the present invention in the field of cosmetics for the treatment of wrinkles.

## Description

The present invention relates to the field of molecular biology, more precisely to molecular biology applied to cosmetics, even more precisely to peptides and compositions comprising said peptides, able to modulate sodium channels providing for a decreased signal and/or expression thereof and, hence, provide for muscle relaxation or less muscle contraction. The peptides and compositions comprising said peptides are also able to maintain and/or synthesize or increase extracellular matrix (ECM) components. Therefore, said peptides and compositions are effective in the treatment of conditions related with the modulation of the above-mentioned aspects (for example, muscle relaxation or maintenance or synthesis of ECM) and are useful in cosmetics (anti-wrinkling activity, activity against sagging or looser skin, improvement of skin firmness and/or elasticity).

In the recent years, there has been a growing interest in the population to prevent, reduce or eliminate skin imperfections, in order to maintain a skin with a healthy and youthful aspect. This has led to an increasing interest in the causes that lead to the appearance of skin imperfections and in the search of compounds and compositions which can prevent or alleviate these imperfections.

Wrinkle (for example, expression wrinkles) formation basis or mechanism is a tension of muscles dragging the skin inwardly. This muscular tension is the result of an hyperactivity of the nerves enervating the corresponding muscles. Said nervous hyperactivity is, in turn, characterized by an uncontrolled and excessive release of neurotransmitters exciting muscular fibers.

In this connection several cosmetic approaches against wrinkles and the above-mentioned generation mechanism have been generated, as, for example:
- Botulinum toxins: they have been widely used with the aim of reducing and/or eliminating expression wrinkles, especially serotype A (BOTOX^{®} Cosmetic, Allergan Inc.). Said botulinum toxins are injected locally and their paralytic effects are reversible with an average duration of 6 months requiring, hence, repeated injections. It is known the risk of triggering an immune reaction against the botulinum preparation leading to a loss of the treatment efficacy. Other serotypes of botulinum toxins, such as B, F and E, have also been considered to overcome this problem, however, said serotypes also have the risk of triggering an immune response. At a molecular level, botulinum toxins are proteases degrading neuronal proteins that are involved in the calcium ion-activated exocytosis mechanism. For example, botulinum toxin A, truncates the neuronal SNAP-25 protein.
- It is also known in the state of the art that certain peptides derived from the sequences of the proteins forming the SNARE complex can inhibit neuronal exocytosis, such as, for example: peptides derived from the amino and carboxyl domains of the SNAP-25 (see, for example, PCT Patent application WO97/34620, European Patent EP1180524B1 and European Patent EP2123673B1); peptides derived from synaptobrevin or from syntaxin (see, for example, PCT Patent application WO97/34620; Blanes-Mira, C., Clemente, J., Jodas, G., Gil, A., Fernandez-Ballester, G., Ponsati, B., Gutierrez, L., Perez-Paya, E., Ferrer-Montiel, A. A synthetic hexapeptide (Argireline) with antiwrinkle activity. International Journal of Cosmetic Science, (2002), 24, 303-310); and peptides competing with specific sequences located in the surface of interaction between Munc18 and Syntaxin-1 (see PCT Patent application PCT/EP2019/054479).
- In the state of the art there is also a conotoxin named Activen XEP-018^{™}, which is a biomimetic peptide that mimics Cone Snail venom (*Conus consors*) from the Indo-West Pacific region and which shows botox-like activity. Biologically, it acts as a modulator of the neuromuscular transmission, as well as a potent and selective blocker of the voltage-gated sodium channel Nav1.4. Cosmetically, it acts as an instant line relaxer giving a smooth skin appearance.

However, there is still the need to find new molecules (preferably, peptides) with anti-wrinkling activity and which can help in the maintenance and synthesis of the ECM.

Voltage-gated sodium channels control nerve signalling through management of action-potential propagation. Inhibition of sodium channels reduces the flow of action potentials along the nerves and thus the effect of the signal. In the skin sodium channels such as NaV1.3 and closely related NaV1.4 are found in the epidermis, subcutaneous adipose tissue, skeletal muscle and nerves. These systems act together in managing skin muscle tone and thus the depth of wrinkles and creases in the skin.

In this sense, it is noted that *Takifugu rubripes* (Japanese puffer, Tiger puffer) is a prized food fish in Japan, despite the liver and ovaries being extremely toxic due to the presence of tetrodotoxin (TTX), a potent toxin that specifically binds to voltage gated sodium channels. TTX binds physically to voltage gated sodium channels and blocks the flow of sodium ions through said channels, thereby preventing action potential (AP) generation and propagation. Additionally, said pufferfish are well known for their resilient skin, able to inflate their bodies to deter predators and recovering their original morphology in a short time once danger is overcome. Therefore, several key complementary activities can be potentially identified in said peptides for human skin which in turn have the potential to have a positive impact against wrinkles, including both, muscle-related or expression wrinkles and chronological wrinkles.

The inventors of the present invention, after extensive and exhaustive research, have surprisingly found that peptides derived from *Takifugu rubripes* (Japanese puffer, Tiger puffer) (from their proteins or genome) and/or peptides derived thereof by means of conservative substitutions provide for the appropriate cosmetic anti-wrinkling activity. Said peptides are capable of providing the required muscle relaxing activity by means of the modulation of voltage-gated sodium channels (decreasing the signal or activity provided by said channels). In addition, the peptides of the present invention have also shown to be able to modulate expression of voltage-gated sodium channels (downregulate or decrease the expression of said channels) and to modulate the release of noradrenaline (decrease the levels and, hence, the release of said neurotransmitter). Moreover, the peptides of the present invention are also able to upregulate the expression of key extracellular matrix (hereinafter, ECM) components, at the gene and protein expression levels, such as collagen I, elastin and hyaluronic acid. Altogether validates the usefulness of the peptides of the present invention in cosmetics for the treatment, prevention and/or reduction of wrinkles (preferably, both, chronological wrinkles and muscle-derived or expression wrinkles); treatment, prevention and/or reduction of sagging or looser skin; treatment, prevention and/or reduction of skin roughness; and improvement of skin firmness and/or elasticity.

Blocking voltage-gated sodium channels (decreasing signaling and/or activity thereof) for muscle relaxation is an interesting mechanism of action which has not yet been widely explored and which is clearly different from the mechanism of action of the products which are currently being widely used in the market. The peptides of the present invention reduce neurotransmission before it arrives at the neuromuscular junction.

In addition, the peptides of the present invention, due to their size have inherent advantages, as, for example, increased or prolonged stability and increased or improved penetration, preferably, through the skin.

In a first aspect, the present invention refers to a peptide of between 4 and 8 amino acids capable of decreasing the signal and/or activity of at least one type of sodium channel and, hence, with anti-wrinkling activity.

In a second aspect, the present invention refers to a composition comprising a peptide of the present invention.

In an additional aspect the present invention refers to the use as a cosmetic of a peptide of the present invention or of a composition of the present invention, in a subject in need of the treatment.

In a fourth aspect, the present invention refers to cosmetic use of a peptide of the present invention or a composition of the present invention in a subject in need of the treatment.

In a final aspect, the present invention refers to a method to reduce, prevent and/or eliminate signs of skin aging in a subject comprising the administration of a peptide of the present invention or a composition of the present invention to the subject.

The term "non-cyclic aliphatic group" and its plural, as used herein, have the common meaning given in the state of the art to said terms. Therefore, these terms refer to, for example and not restricted to, linear or branched alkyl, alkenyl and alkynyl groups.

The term "alkyl group" and its plural, as used herein, refer to a saturated, linear or branched group, which has between 1 and 24, preferably between 1 and 16, more preferably between 1 and 14, even more preferably between 1 and 12, and even more preferably still between 1, 2, 3, 4, 5 or 6 carbon atoms and which is bound to the rest of the molecule by a simple bond, including, for example and not restricted to, methyl, ethyl, isopropyl, n-propyl, i-propyl, isobutyl, tert-butyl, n-butyl, sec-butyl, n-pentyl, n-hexyl, heptyl, octyl, decyl, dodecyl, lauryl, hexadecyl, octadecyl, amyl, 2-ethylhexyl, 2-methylbutyl, 5-methylhexyl and similar. The alkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkenyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the vinyl, oleyl, linoleyl and similar groups. The alkenyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkynyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the ethinyl group, 1-propinyl, 2-propinyl, 1-butinyl, 2-butinyl, 3-butinyl, pentinyl, such as 1-pentinyl and similar groups. The alkynyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alicyclic group" and its plural, as used herein, have the common meaning given in the state of the art to said terms. Hence, these terms are used to refer to, for example and not restricted to, cycloalkyl or cycloalkenyl or cycloalkynyl groups.

The term "cycloalkyl" and its plural, as used herein, refer to a saturated mono- or polycyclic aliphatic group which has between 3 and 24, preferably between 3 and 16, more preferably between 3 and 14, even more preferably between 3 and 12, even more preferably still 3, 4, 5 or 6 carbon atoms and which is bound to the rest of the molecule through a single bond, including, for example and not limited to, cyclopropyl, cyclobutyl, cyclopentyl, cyclohexyl, cycloheptyl, methyl cyclohexyl, dimethyl cyclohexyl, octahydroindene, decahydronaphthalene, dodecahydro-phenalene, adamantyl and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkenyl" and its plural, as used herein, refer to a non-aromatic mono- or polycyclic aliphatic group which has between 5 and 24, preferably between 5 and 16, more preferably between 5 and 14, even more preferably between 5 and 12, even more preferably still 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the cyclopent-1-en-1-yl group and similar groups, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkynyl" and its plural, as used herein, refer to a non-aromatic mono- or polycyclic aliphatic group which has between 8 and 24, preferably between 8 and 16, more preferably between 8 and 14, even more preferably between 8 and 12, even more preferably still 8 or 9 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the cyclooct-2-yn-1-yl group and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "aryl group" and its plural, as used herein, refer to an aromatic group which has between 6 and 30, preferably between 6 and 18, more preferably between 6 and 10, even more preferably 6 or 10 carbon atoms, which comprises 1, 2, 3 or 4 aromatic rings, bound by a carbon-carbon bond or fused, and which is bound to the rest of the molecule through a single bond, including, for example and not restricted to, phenyl, naphthyl, diphenyl, indenyl, phenanthryl or anthranyl among others. The aryl group can be optionally substituted by one or more substituents, such as, halo, hydroxy, alkoxy, carboxy, carbonyl, cyano, acyl, alkoxy-carbonyl, amino, nitro, mercapto and alkoxythio.

The term "aralkyl group" and its plural, as used herein, refer to an alkyl group substituted by an aromatic group, with between 7 and 24 carbon atoms and including, for example and not restricted to, -(CH₂)1-6-phenyl, -(CH₂)1-6-(1-naphtyl), -(CH₂)1-6-(2-naphtyl),-(CH₂)1-6-CH(phenyl)₂ and similar. The aralkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heterocyclic group" and its plural, as used herein, refer to a 3-10 member heterocycyl or hydrocarbon ring, in which one or more of the ring atoms, preferably 1, 2 or 3 of the ring atoms, is a different element to carbon, such as nitrogen, oxygen or sulfur and may be saturated or unsaturated. For the purposes of this invention, the heterocyclyl can be a cyclic, monocyclic, bicyclic or tricyclic system which may include fused ring systems; and the nitrogen, carbon or sulfur atoms can be optionally oxidized in the heterocyclyl radical; the nitrogen atom can optionally be quaternized; and the heterocyclyl radical may be partially or completely saturated or may be aromatic. With increasing preference, the term heterocyclic relates to a 5 or 6-member ring. The heterocyclic groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heteroarylalkyl group" and its plural, as used herein, refer to an alkyl group substituted with a substituted or unsubstituted aromatic heterocyclyl group, the alkyl group having from 1 to 6 carbon atoms and the aromatic heterocyclyl group between 2 and 24 carbon atoms and from 1 to 3 atoms other than carbon and including, for example and not restricted to, -(CH₂)1-6-imidazolyl, -(CH₂)1-6-triazolyl, -(CH₂)1-6-thienyl,-(CH₂)1-6-furyl, -(CH₂)1-6-pyrrolidinyl and similar. The heteroarylalkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The terms "halo" or "halogen", as used in the present document, refer to fluorine, chlorine, bromine or iodine, and its anions are referred to as halides.

As used herein, the term "derivative" and its plural, refer to cosmetically acceptable compounds, this is, derived from the compound of interest that can be used in the preparation of a cosmetic, and to cosmetically unacceptable derivatives since these may be useful in the preparation of cosmetically acceptable derivatives.

As used in the present document, the term "salt" and its plurals refer to any type of salt from among those known in the state of the art, for example, halide salts, hydroxy acid salts (such as oxyacid salts, acid salts, basic salts and double salts), hydroxo salts, mixed salts, oxy salts or other hydrated salts. This term comprises cosmetically acceptable salts; and cosmetically unacceptable salts, since the latter may be useful in the preparation of cosmetically acceptable salts.

As used in the present document, the term "isomer" and its plural refer to optical isomers, enantiomers, stereoisomers or diastereoisomers. The individual enantiomers or diastereoisomers, as well as their mixtures, may be separated by conventional techniques known in the state of the art.

As used herein, the term "solvate" and its plural refer to any solvate known in the state of the art, such as polar, apolar or amphiphilic solvates, and include any cosmetically acceptable solvate which, when administered or applied to the interested subject (directly or indirectly) provides the compound of interest (the peptide or peptides of the present invention). Preferably, the solvate is a hydrate, a solvate with an alcohol such as methanol, ethanol, propanol or isopropanol, a solvate with an ester such as ethyl acetate, a solvate with an ether such as methyl ether, ethyl ether or THF (tetrahydrofuran) or a solvate with DMF (dimethylformamide), and more preferably a hydrate or a solvate with an alcohol such as ethanol.

In addition, as used herein, the term "amino acid" and its plural include the amino acids codified by the genetic code as well as uncodified amino acids, whether they are natural or not and whether they are D- and L-amino acids. Examples of uncodified amino acids are, without restriction, citrulline, ornithine, sarcosine, desmosine, norvaline, 4-aminobutyric acid, 2-aminobutyric acid, 2-aminoisobutyric acid, 6-aminohexanoic acid, 1-naphthylalanine, 2-naphthylalanine, 2-aminobenzoic acid, 4 aminobenzoic acid, 4-chlorophenylalanine, 2,3-diaminopropionic acid, 2,4 diaminobutyric acid, cycloserine, carnitine, cysteine, penicillamine, pyroglutamic acid, thienylalanine, hydroxyproline, allo-isoleucine, allo-threonine, isonipecotic acid, isoserine, phenylglycine, statin, β-alanine, norleucine, N-methylamino acids, α-amino acids and β-amino acids, among others, as well as their derivatives. Nevertheless, further unnatural amino acids are known in the state of the art (see, for example, "Unusual amino acids in peptide synthesis" by D. C. Roberts and F. Vellaccio, The Peptides, Vol. 5 (1983), Chapter VI, Gross E. and Meienhofer J., Eds., Academic Press, New York, USA).

The "percentage of identity" regarding peptides, polypeptides and proteins, as used herein, has the meaning commonly attributed in the state of the art and, hence, relates to the percentage of amino acids which are identical between two amino acid sequences which are compared after an optimal alignment of these sequences, where said percentage is merely statistical and the differences between the two amino acid sequences are randomly distributed throughout the sequence. "Optimal alignment" is understood as that alignment of amino acid sequences giving rise to a greater percentage of identity. The percentage of identity is calculated by determining the number of identical positions in which an amino acid is identical in the two compared sequences, dividing the number of identical positions by the number of compared positions and multiplying the result obtained by 100 to obtain the percentage of identity between the two sequences. The sequence comparisons between two amino acid sequences can be carried out manually or by means of computer programs known in the state of the art, such as the BLAST (Basic Local Alignment Search Tool) algorithm.

As noted above, in a first aspect, the present invention refers to a peptide of between 4 and 8 amino acids (this is, a peptide that comprises between 4 and 8 amino acids, more preferably a peptide that has between 4 and 8 amino acids, more preferably a peptide consisting essentially of between 4 and 8 amino acids, more preferably, a peptide consisting of between 4 and 8 amino acids) capable of decreasing the signal and/or activity of at least one type of sodium channel; its acceptable isomers, salts, solvates derivatives and/or mixtures thereof.

As it has been noted above, the peptide of the present invention is preferably derived from a protein of puffer fish and/or from the genome of puffer fish. The puffer fish can be any puffer fish known now or discovered in the future. More preferably, the puffer fish is *Takifugu rubripes* (Japanese puffer, Tiger puffer). The peptide of the present invention can also be a peptide obtained or derived from a peptide derived from a protein of puffer fish and/or from the genome of puffer fish, preferably, by means of conservative substitutions.

It is contemplated that the amino acids used or present in the peptides of the present invention are L-amino acids, D-amino acids or combinations thereof. In a preferred embodiment, the amino acids used or present in the peptides of the present invention are L-amino acids.

Preferably, the isomers mentioned above are stereoisomers. It is contemplated that said stereoisomers are enantiomers or diastereoisomers. Hence, in a preferred embodiment of the present invention, the peptide is a racemic mixture, a diastereomeric mixture, a pure enantiomer or a pure diastereoisomer.

In addition, isomers, salts, solvates, derivatives and/or mixtures thereof are, preferably, cosmetically acceptable isomers, salts, solvates, derivatives and/or mixtures thereof.

As can be directly derived from the examples included below, the peptides of the present invention provide for a modulation sodium channels, which shows up as a decrease in the signal and/or activity of at least one type of sodium channel. Said decrease in the activity and/or signal of at least one type of sodium channel is, preferably a decrease in the current provided by said at least one type of sodium channel, this is, a decrease in the action potential.

The sodium channels mentioned above are, preferably, voltage-gated sodium channels.

The at least one type of sodium channel can be any type of sodium channel. Examples of sodium channels are NaV1.1, NaV1.2, NaV1.3, NaV1.4, NaV1.5, NaV1.6, NaV1.7, NaV1.8 and NaV1.9. It is contemplated that the peptide of the present invention is capable of decreasing the activity and/or the signal of one type of sodium channel or several types (two or more) of sodium channels.

In an embodiment, the peptide of the present invention decreases the activity and/or the signal, at least, of the sodium channel Nav1.3, more preferably the peptide of the invention only decreases the activity and/or the signal of the sodium channel NaV1.3.

In a further embodiment, the peptide of the present invention decreases the activity and/or the signal, at least, of the sodium channel NaV1.4, more preferably the peptide of the invention only decreases the activity and/or the signal of the sodium channel NaV1.4.

In a preferred embodiment, the peptide of the present invention decreases the signal and/or the activity, at least, of the sodium channels Nav1.3 and NaV1.4, more preferably the peptide of the invention only decreases the activity and/or the signal of the sodium channels NaV1.3 and NaV1.4.

It is noted that the peptide of the present invention is capable of decreasing the activity and/or the signal of the at least one type of sodium channel wherever said sodium channels are present. Preferably, the peptide of the present invention decreases the activity and/or the signal of the at least one sodium channel at a presynaptic level, preferably in a neuron.

In some embodiments of the present invention, decreasing the signal and/or the activity of at least one type of sodium channel means blocking at least one type of sodium channel, which can be a partial blocking or a total blocking, more preferably a partial blocking.

In addition, in some embodiments, which can be combined with any of the embodiments mentioned above, the peptide of the present invention is also capable of modulating the expression of at least one type of sodium channel, more preferably, downregulating the expression of at least one type of sodium channel. The at least one type of sodium channel is as explained above. In these embodiments, the peptide of the present invention is capable of downregulating the expression of at least one type of sodium channel wherever said sodium channels are expressed. Preferably, the peptide of the present invention downregulates the expression of the at least one sodium channel at a postsynaptic level, preferably in a muscle cell, more preferably in a skeletal muscle cell.

In addition, in some embodiments, which can be combined with any of the embodiments mentioned above, the peptide of the present invention is also capable of maintaining and/or synthesizing ECM, more precisely of promoting maintenance of the ECM and/or promoting and/or increasing synthesis of ECM components. The peptides of the present invention perform said activities by upregulating the expression of relevant genes and proteins from the ECM; more preferably, the peptide of the present invention is also capable of upregulating at least one gene and/or protein in charge of the synthesis and/or of the maintenance of the ECM; more preferably, the peptide of the present invention is also capable of upregulating at least one gene and/or protein in charge of the synthesis and/or of the maintenance of the ECM selected from collagen I, elastin, hyaluronic acid or combinations thereof; even more preferably, the peptide of the present invention is also capable of upregulating collagen I, elastin and hyaluronic acid, all at a genetic and/or protein level.

In a preferred embodiment, which can be combined with any of the embodiments mentioned above, the peptide of the present invention is capable of increasing the synthesis (and, hence, the levels thereof) of elastin, procollagen I alpha 1 and hyaluronic acid, at a protein level.

In a further preferred embodiment, which can be combined with any of the embodiments mentioned above, the peptide of the present invention is capable of downregulating the expression of genes which provide for the degradation of the ECM and/or upregulate genes which provide for the maintenance or synthesis of the ECM. More preferably, the downregulated genes are selected from MMP1, HYAL1 or combinations thereof; and the upregulated genes are selected from TIMP2, HAS1, FBLN5, LOXL1, COL4A1, COL3A1, COL7A1 or combinations thereof. Even more preferably, the downregulated genes are MMP1 and HYAL1 and the upregulated genes are TIMP2, HAS1, FBLN5, LOXL1, COL4A1, COL3A1 and COL7A1.

The peptides of the present invention are capable of performing the above-mentioned activity of maintaining and/or synthesizing ECM (more preferably, of promoting maintenance of the ECM and/or promoting and/or increasing synthesis of ECM) at least in fibroblasts.

In further embodiments, which can be combined with any of the embodiments mentioned above, the peptide of the present decreases the level or quantity of at least one neurotransmitter, more preferably noradrenaline. Said decrease of the levels of the at least one neurotransmitter is preferably performed at a presynaptic level, preferably in a neuron. As it is readily derivable, the decrease of the levels or quantity of the at least one neurotransmitter will lead to a decrease or an inhibition in the secretion of said neurotransmitter.

The above activities have been demonstrated in the examples included below and provide for the ability of the peptides of the present invention to induce or produce muscle relaxation (or avoid muscle contraction or excessive muscle contraction) which in turn provides for an anti-wrinkling effect (preferably, for expression wrinkles). In addition, the above activities also validate the usefulness of the peptides of the present invention for prevention and/or reduction of chronological wrinkles (preferably, their depth, length and/or roughness); treatment, prevention and/or reduction of sagging or looser skin; as well as an improvement of skin firmness and elasticity.

In a preferred embodiment, which can be combined with any of the embodiments mentioned above, the peptide of the present invention is a peptide of 5 or 6 amino acids (this is, a peptide that comprises 5 or 6 amino acids, more preferably a peptide that has 5 or 6 amino acids, more preferably a peptide consisting essentially of 5 or 6 amino acids, more preferably, a peptide consisting of 5 or 6 amino acids).

In addition, preferably, the peptide of the present invention, in any of the embodiments explained above, comprises at least one charged amino acid (positively or negatively). Even more preferably, the amino acid of the first position and/or of the second position is a charged amino acid (positively or negatively). Still more preferably, in the peptide of the present invention, the first three amino acids are charged amino acids (positively or negatively).

In a preferred embodiment, which can be combined with any of the embodiments mentioned above, in the peptide of the present invention the amino acids in the first and in the third position are positively charged amino acids and the amino acid in the second position is a positively or negatively charged amino acid.

Moreover, preferably, in any of the above embodiments, in the peptide of the present invention, the rest of the amino acids are polar without charge, apolar and/or aromatic. In addition, preferably, if present, the amino acid in the fifth position is aromatic, preferably selected from Tyr or Trp. Also preferably, if present, the amino acid in the sixth position is polar without charge or apolar.

Therefore, in a preferred embodiment, the peptide of the present invention comprises at least one aromatic amino acid, more preferably, 1 or 2 aromatic amino acids. Even more preferably:
- If the peptide of the present invention has 5 amino acids it has one aromatic amino acid, more preferably in the fifth position. In this case the aromatic amino acid is preferably Tyr.
- If the peptide of the present invention has 6 amino acids, it has 2 aromatic amino acids, one in the fourth position and another one in the fifth position. In this case the aromatic amino acids are selected from Tyr, Trp, Phe or combinations thereof.

In any of the above embodiments, the charged amino acid (positively or negatively) is, preferably, selected from Lys, Arg, His, Asp.

Therefore, in a preferred embodiment, in the peptide of the present invention the charged amino acid in position 1 is Lys. Even more preferably, in the peptide of the present invention the first three amino acids of the peptide are: Lys-Arg-Arg, Lys-His-Arg, Lys-Asp-Arg or Lys-Lys-His.

In a preferred embodiment, the peptide of the present invention has the sequence of formula (I):
R₁-AA1-AA2-AA3-AA4-AA5-AA6-R₂
(R₁-SEQ ID NO:7-R₂)
wherein
AA1 is selected from Lys, Arg or His;
AA2 is selected from Lys, Arg, His, Asp or Glu;
AA3 is selected from Lys, Arg or His;
AA4 is selected from Gly, Ala, Val, Leu, Met, Ile, Ser, Thr, Cys, Pro, Asn, Gln, Phe, Tyr or Trp;
AA5 is selected from Phe, Tyr or Trp;
AA6 is absent or is selected from Gly, Ala, Val, Leu, Met, Ile, Ser, Thr, Cys, Pro, Asn or Gln;
R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, R₁ and R₂ are absent and AA1 is directly bound to the last amino acid of the peptide, this is, AA6 or AA5 in case AA6 is absent (forming a cyclic peptide).

R₁ is linked to the N-terminal of the peptide, this is, to the amino group of AA1 (it substitutes or replaces an H from the amino group of AA1).

R₂ is linked to the C-terminal of the peptide, this is, to the carboxy group of AA6 or to the carboxy group of AA5 in case AA6 is absent (it substitutes or replaces the OH of the carboxy group).

Preferably, in formula (I):
AA1 is Lys
AA2 is selected from Arg, His, Lys or Asp;
AA3 is selected from Arg or His;
AA4 is selected from Tyr, Ser, Val or Phe;
AA5 is selected from Tyr or Trp;
AA6 is absent or is selected from Cys or Ala.

In a preferred embodiment, the peptide of the present invention is selected from:
- R₁-Arg-Trp-Gln-Lys-Asn-Gln-R₂ (R₁-SEQ ID NO: 1-R₂);
- R₁-Lys-Arg-Arg-Tyr-Tyr-Cys-R₂ (R₁-SEQ ID NO: 2-R₂); or
- R₁-Lys-His-Arg-Ser-Tyr-R₂ (R₁-SEQ ID NO: 3-R₂);
wherein:
R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, R₁ and R₂ are absent and AA1 is directly bound to the last amino acid of the peptide (forming a cyclic peptide).

Also in a preferred embodiment, the peptide of the present invention is selected from:
- R₁-Lys-Asp-Arg-Val-Tyr-R₂ (R₁-SEQ ID NO: 4-R₂);
- R₁-Ile-Glu-Pro-Tyr-Tyr-Val-R₂ (R₁-SEQ ID NO: 5-R₂); or
- R₁-Lys-Lys-His-Phe-Trp-Ala-R₂ (R₁-SEQ ID NO: 6-R₂);
and wherein:
R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, R₁ and R₂ are absent and AA1 is directly bound to the last amino acid of the peptide (forming a cyclic peptide).

More preferably, the peptide of the present invention is selected from:
- R₁-Lys-Asp-Arg-Val-Tyr-R₂ (R₁-SEQ ID NO: 4-R₂); or
- R₁-Lys-Lys-His-Phe-Trp-Ala-R₂ (R₁-SEQ ID NO: 6-R₂);
being R₁ and R₂ as disclosed above.

Even more preferably, the peptide of the present invention is:
- R₁-Lys-Asp-Arg-Val-Tyr-R₂ (R₁-SEQ ID NO: 4-R₂);
being R₁ and R₂ as disclosed above.

In any of the above embodiments, preferably, R₁ is H or acetyl, more preferably R₁ is H.

Also, in any of the above embodiments, R₂ is NH₂ or OH, more preferably R₂ is NH₂.

Hence, in the most preferred embodiment, the peptide of the present invention is selected from:
- Arg-Trp-Gln-Lys-Asn-Gln-NH₂ (SEQ ID NO: 1-NH₂);
- Lys-Arg-Arg-Tyr-Tyr-Cys-NH₂ (SEQ ID NO: 2-NH₂);
- Lys-His-Arg-Ser-Tyr-NH₂ (SEQ ID NO: 3-NH₂);
- Lys-Asp-Arg-Val-Tyr-NH₂ (SEQ ID NO: 4-NH₂);
- Ile-Glu-Pro-Tyr-Tyr-Val-NH₂ (SEQ ID NO: 5-NH₂); or
- Lys-Lys-His-Phe-Trp-Ala-NH₂ (SEQ ID NO: 6-NH₂)

Even more preferably, the peptide of the present invention is:
- Lys-Asp-Arg-Val-Tyr-NH₂ (SEQ ID NO: 4-NH₂); or
- Lys-Lys-His-Phe-Trp-Ala-NH₂ (SEQ ID NO: 6-NH₂)
even more preferably:
Lys-Asp-Arg-Val-Tyr-NH₂ (SEQ ID NO: 4-NH₂).

It is noted that peptides SEQ ID NO:1 to SEQ ID NO: 3 are directly derived from proteins and/or the genome of the puffer fish *Takifugu rubripes* (Japanese puffer, Tiger puffer). More precisely:
- SEQ ID NO: 1 is derived from the sequence of Nucleolar complex protein 2 homolog of the puffer fish *Takifugu rubripes* (Uniprot reference number: H2TGB2, amino acids 88 to 93).
- SEQ ID NO: 2 is derived from the sequence of Disabled homolog2-interacting protein-like isoform X2 (Synaptic Ras-GTPase activating domain) (NCBI Reference Sequence: XP_003976523.1, amino acids 64 to 68).
- SEQ ID NO: 3 is derived from a genome sequence predicted as calcium-independent phospholipase A2-gamma-like isoform X2 (NCBI Reference Sequence: XP_011605802.2, amino acids 7 to 11).

Peptides of SEQ ID NO: 4 to 6 are derived from peptides of SEQ ID NO: 1 to 3 by means of conservative substitutions.

Within the scope of the present invention are also included peptides derived or obtained from any of the peptides disclosed above, preferably incorporating one or more conservative substitutions, as long as they retain or show or improve at least one of the activities mentioned above (and, preferably, all of the activities mentioned above for the peptides of the present invention).

In addition, also included within the scope of the present invention are peptides with a 70% percentage identity, preferably 80%, more preferably 90%, more preferably 95%, even more preferably 99% percentage identity with any of the specific sequences mentioned above as long as they retain or show or improve at least one of the activities mentioned above (and, preferably, all of the activities mentioned above for the peptides of the present invention).

As it is directly derivable from the above, it is contemplated that the peptide of the present invention is a linear peptide or a cyclic peptide.

In an embodiment, which can be combined with any of the above embodiments, the peptide is cyclic. In this case, the first and the last amino acid of the peptide of the present invention (in accordance with the above explanation) are bound. Therefore, appropriate modification to any of the above embodiments has to be done (for example, R₁ and R₂ may be absent as the amino acids to which said moieties are bound, are now bound to each other).

In a preferred embodiment, which can be combined with any of the above embodiments, the peptide is linear.

The peptides of the present invention may be synthesized and produced by any means known in the state of the art. For example, they may be synthesized and produced by chemical synthesis (preferably, by means of solid phase peptide synthesis), expressing said peptides in cell cultures or by means of transgenic production of the peptide in plants or animals. In addition, the peptides of the present invention may be purified by any means known in the state of the art.

In a preferred embodiment, which can be combined with any of the embodiments disclosed above, the peptide of the present invention is suited or adapted to be applied by means of iontophoresis, more preferably, in the face of a subject (preferably, a human).

In a further preferred embodiment, which can be combined with any of the embodiments disclosed above, the peptide of the present invention is suited or adapted to be applied subcutaneously, more preferably, in the face of a subject (preferably, a human).

In another preferred embodiment, which can be combined with any of the embodiments disclosed above, the peptide of the present invention is suited or adapted to be applied topically (preferably, in the form of a cream), more preferably, in the face of a subject (preferably, a human).

As it is directly derivable from the examples included below, the peptides of the present invention have the required activities to provide for the prevention, treatment and/or reduction of wrinkles at least by means of muscle relaxation as well as by means of ECM maintenance and synthesis. In addition, the peptides of the present invention also have the required activities to provide for the prevention, treatment and/or reduction of looser or sagging skin and skin roughness (preferably, wrinkle roughness); as well as for the improvement of skin firmness and elasticity. Therefore, the peptides of the present invention solve the technical problem mentioned above and are useful in cosmetics.

In a second aspect, the present invention refers to a composition comprising a peptide of the present invention.

The peptide of the present invention is as disclosed above in the first aspect of the present invention.

The composition of the present invention is preferably a cosmetic composition. Hence, in this embodiment, the cosmetic composition of the present invention comprises a cosmetically effective amount of the at least one peptide of the present invention, more preferably the cosmetic composition of the present invention comprises from 0.0001% (weight/volume in g/100mL, hereinafter, w/v) to 0.05% (w/v) of a peptide of the present invention. In a most preferred embodiment, the cosmetic composition of the present invention comprises from 0.0001% (w/v) to 0.005% (w/v) of a peptide of the present invention, more preferably, from 0.0005% (w/v) y 0.0015% (w/v).

It is contemplated that the cosmetic composition of the present invention also comprises at least one additional cosmetic ingredient. Said additional cosmetic ingredient can be at least one excipient and/or at least one additional cosmetic active ingredient. Said additional cosmetic ingredient can be any cosmetic ingredient known in the state of the art as long as it does not affect or it does not affect in an unacceptable manner the activity of the peptides of the present invention.

In an embodiment, the composition of the present invention consists essentially of peptides of the present invention.

In another embodiment, the composition of the present invention consists of peptides of the present invention.

In a third aspect, the present invention refers to the use as a cosmetic of a peptide of the present invention or of a composition of the present invention, in a subject in need of the treatment.

The peptide of the present invention is as disclosed above in the first aspect of the present invention.

The composition of the present invention is as disclosed above in the second aspect of the present invention.

The peptide or the composition of the present invention are used in a cosmetically effective amount or quantity.

In a preferred embodiment, the use as a cosmetic is to reduce, prevent and/or eliminate signs of skin aging.

Preferably, the signs of skin aging are wrinkles, skin roughness (preferably, wrinkle roughness), looser skin or sagging skin, more preferably, wrinkles, skin roughness, non-inflammatory looser skin or non-inflammatory sagging skin, more preferably, wrinkles, even more preferably facial wrinkles. Said wrinkles can be chronological wrinkles or expression (muscle-derived) wrinkles.

Non-inflammatory looser skin is equivalent to cosmetic looser skin, this is, non-pathological (non-medical condition).

Non-inflammatory sagging skin is equivalent to cosmetic sagging skin, this is, non-pathological (non-medical condition).

As it is directly derivable from what it has been stated above, in the present invention, signs of skin aging (preferably, wrinkles, both chronological and expression wrinkles) are prevented, reduced and/or eliminated (by the peptide of the present invention or by the composition of the present invention) by means of the blockage of the neuromuscular contraction as already explained above, and/or by means of maintaining or synthesising ECM.

The at least one sodium channel and the at least one neurotransmitter are as explained above in the first aspect of the present invention.

In this third aspect of the present invention, the subject in need of the treatment is preferably a mammal, more preferably, a human.

In this third aspect of the present invention it is also contemplated that the use as a cosmetic is for the improvement of skin firmness and/or elasticity.

In a fourth aspect, as noted above, the present invention refers to the cosmetic use of a peptide of the present invention or a composition of the present invention in a subject in need of the treatment.

Preferably, in this fourth aspect of the present invention, the cosmetic use is to reduce, prevent and/or eliminate signs of skin aging.

In this fourth aspect of the present invention it is also contemplated that the cosmetic use is for the improvement of skin firmness and/or elasticity.

The peptide of the present invention is as disclosed above in the first aspect of the present invention.

The composition of the present invention is as disclosed above in the second aspect of the present invention.

The signs of skin aging and the subject in need of the treatment are as explained above in the third aspect of the present invention.

In addition, all the embodiments explained above in the third aspect of the present invention are directly applicable to this fourth aspect of the present invention, with the appropriate adaptations.

In a fifth and final aspect, the present invention refers to a method to reduce, prevent and/or eliminate signs of skin aging in a subject comprising the administration of a peptide of the present invention or a composition of the present invention to the subject.

The peptide of the present invention is as disclosed above in the first aspect of the present invention.

The composition of the present invention is as disclosed above in the second aspect of the present invention.

The signs of skin aging and the subject are as explained above in the third aspect of the present invention.

In addition, all the embodiments explained above in the third and fourth aspects of the present invention are directly applicable to this fifth aspect of the present invention, with the appropriate adaptations.

### SEQUENCE LISTING FREE TEXT

SEQ ID NO: 1
   <223> Peptide derived from the sequence of Nucleolar complex protein 2 homolog of the puffer fish Takifugu rubripes (Uniprot reference number: H2TGB2, amino acids 88 to 93).
SEQ ID NO: 2
   <223> Peptide derived from the sequence of Disabled homolog 2-interacting protein-like isoform X2 (Synaptic Ras-GTPase activating domain) (NCBI Reference Sequence: XP_003976523.1, amino acids 64 to 68).
SEQ ID NO: 3
   <223> Peptide derived from a genome sequence predicted as calcium-independent phospholipase A2-gamma-like isoform X2 (NCBI Reference Sequence: XP_011605802.2, amino acids 7 to 11)
SEQ ID NO: 4
   <223> Artificially synthesized sequence
SEQ ID NO: 5
   <223> Artificially synthesized sequence
SEQ ID NO: 6
   <223> Artificially synthesized sequence
SEQ ID NO: 7
   <223> Artificially synthesized sequence
   <223> Xaa is selected from Lys, Arg or His
   <223> Xaa is selected from Lys, Arg, His, Asp or Glu
   <223> Xaa is selected from Lys, Arg or His
   <223> Xaa is selected from Gly, Ala, Val, Leu, Met, Ile, Ser, Thr, Cys, Pro, Asn, Gln, Phe, Tyr or Trp
   <223> Xaa is selected from Phe, Tyr or Trp
   <223> Xaa is absent or is selected from Gly, Ala, Val, Leu, Met, Ile, Ser, Thr, Cys, Pro, Asn or Gln

To allow a better understanding, the present invention is described in more detail below with reference to the enclosed drawings, which are presented by way of example, and with reference to illustrative and non-limitative examples.

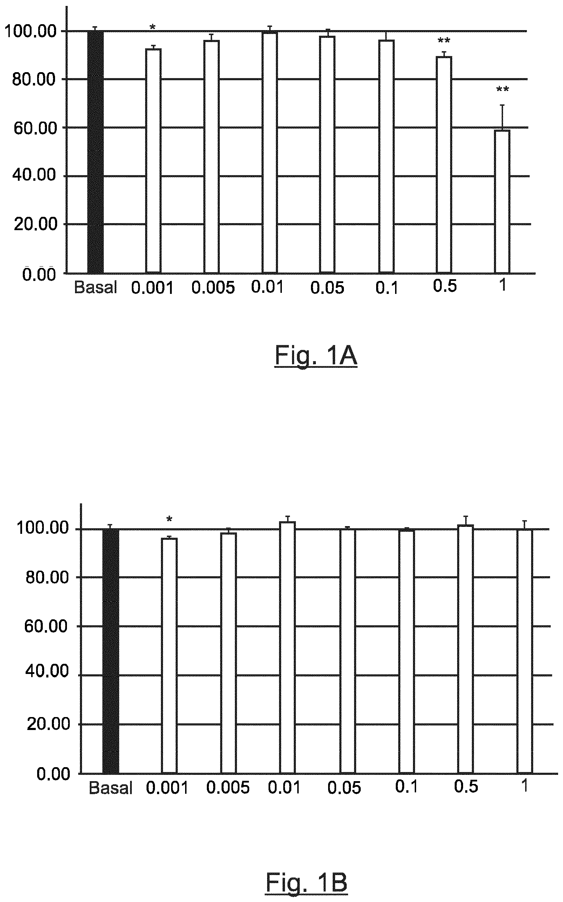

Figure 1 shows the results for cell viability obtained for peptide SEQ ID NO: 4-NH₂ in Example 2 included below. More specifically, Figure 1A shows the viability in HEKa cells and Figure 1B shows the viability in HDFa cells. In both figures the y-axis refers to the percentage of cell viability taking as a 100% the viability of the negative controls (untreated cells). In both figures, the columns in the x-axis, from left to right refer to: negative control (untreated cells); and cells treated with 0.001 mg/mL, 0.005 mg/mL, 0.01 mg/mL, 0.05 mg/mL, 0.1 mg/mL, 0.5 mg/mL or 1 mg/mL of the peptide SEQ ID NO: 4-NH₂.

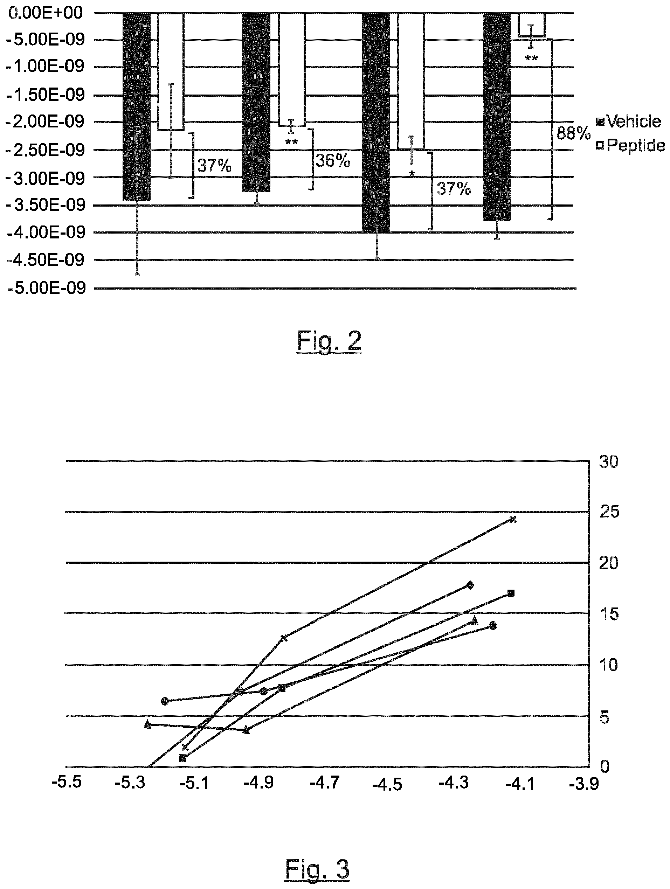

Figure 2 shows the results obtained in Example 3. This is, the obtained current overtime (area under the response curve) for the tested samples and, hence, the activity of voltage-gated sodium channels (NaV1.3). The y-axis refers to the inward current in Amps. The x-axis refers to the different experimental groups tested, for each pair of columns, the one on the left refers to the negative control and the one on the right refers to the treated sample. In the x-axis, the pairs of columns, from left to right refer to the following experimental treatments or groups: SEQ ID NO: 1-NH₂, SEQ ID NO: 2-NH₂, SEQ ID NO: 3-NH₂ and Amitriptyline.
Figure 3 shows the results obtained in Example 4. This figure shows the percentage of inhibition of voltage-gated sodium channels. The y-axis reflects the percentage of inhibition of voltage-gated sodium channels with regard to the negative control (0%) and the x-axis refers to the log of the concentration in M of the different peptides tested. The line with triangles is SEQ ID NO: 2-NH₂; the line with squares is SEQ ID NO: 3-NH₂; the line with crosses is SEQ ID NO: 4-NH₂; the line with circles is SEQ ID NO: 5- NH₂; and the line with rhombus SEQ ID NO: 6- NH₂.

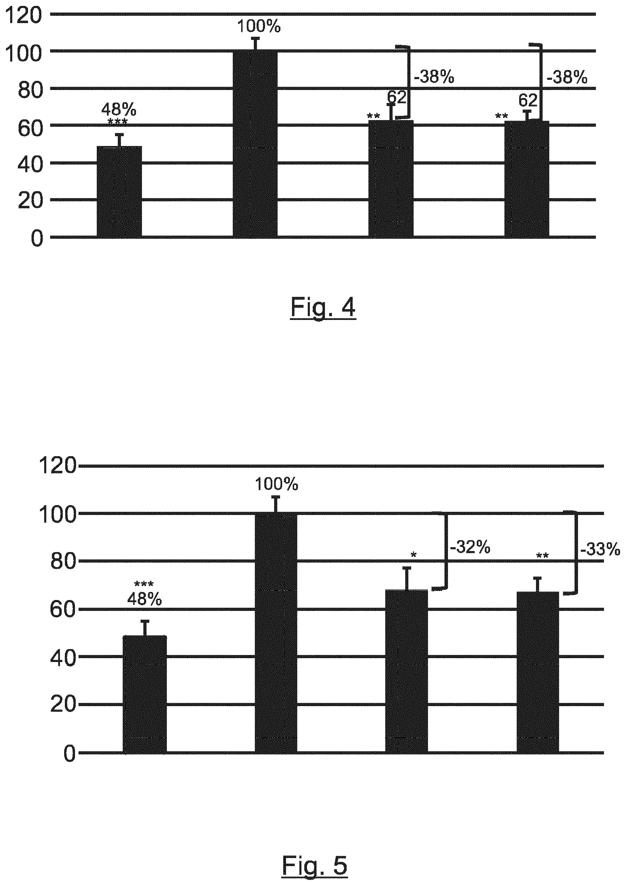

Figure 4 shows the results obtained for the levels of noradrenaline in example 5 in the treatment with SEQ ID NO: 6-NH₂. The y-axis shows the percentage of noradrenaline with regard to the positive control (depolarized cells without treatment, established as 100%). The x-axis shows the different treatment groups, being the bars from left to right: basal state; positive control (depolarized cells without treatment); depolarized cells treated with 0.05 mg/mL of peptide SEQ ID NO: 6-NH₂; and depolarized cells treated with 0.1 mg/mL of peptide SEQ ID NO: 6-NH₂.
Figure 5 shows the results obtained for the levels of noradrenaline in example 5 in the treatment with SEQ ID NO: 4-NH₂. The y-axis shows the percentage of noradrenaline with regard to the positive control (depolarized cells without treatment, established as 100%). The x-axis shows the different treatment groups, being the bars from left to right: basal state; positive control (depolarized cells without treatment); depolarized cells treated with 0.05 mg/mL of peptide SEQ ID NO: 4-NH₂; and depolarized cells treated with 0.1 mg/mL of peptide SEQ ID NO: 4-NH₂.

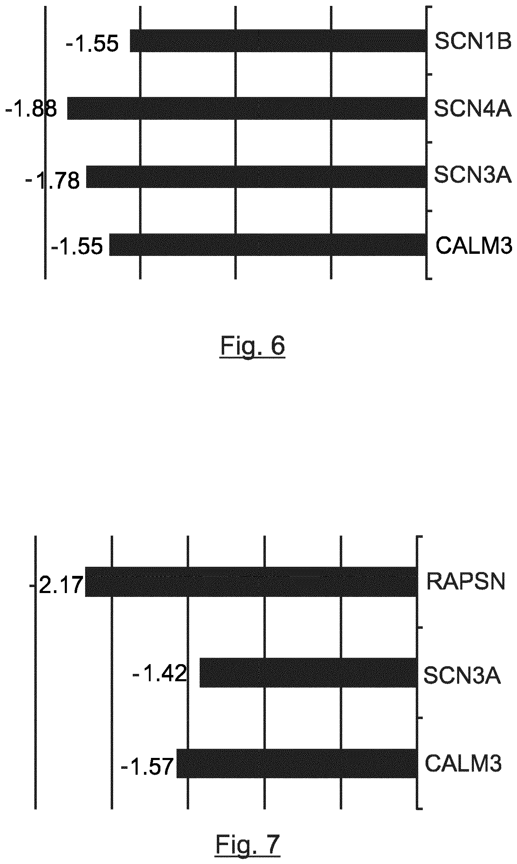

Figure 6 shows the gene expression results obtained in example 6 for the treatment with peptide SEQ ID NO: 4-NH₂ at a concentration of 0.01 mg/mL for 24 hours. Each of the bars refers to one gene, from top to bottom: SCN1B, SCN4A, SCN3A and CALM3. Bars growing towards the left (this is, with a negative value) show a downregulation of the gene.
Figure 7 shows the gene expression results obtained in example 6 for the treatment with peptide SEQ ID NO: 4-NH₂ at a concentration of 0.005 mg/mL for 24 hours. Each of the bars refers to one gene, from top to bottom: RAPSN, SCN3A and CALM3. Bars growing towards the left (this is, with a negative value) show a downregulation of the gene.

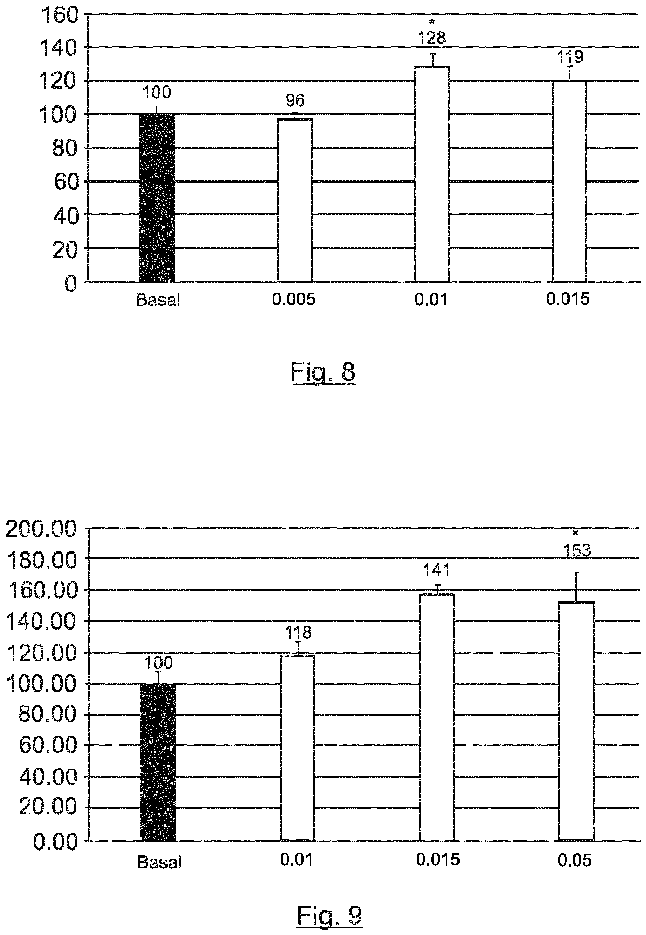

Figure 8 shows the elastin production or synthesis results obtained in example 7 for the treatment of HDFa cells with peptide SEQ ID NO: 4-NH₂ at a concentration of 0.005, 0.01 or 0.015 mg/mL for 48 hours. Elastin production or synthesis is expressed as a percentage with regard to the basal condition (untreated cells) which is established as 100%. The y-axis shows the percentage of elastin with regard to the basal condition. The x-axis shows the different treatment groups, being the bars from left to right: basal condition (untreated cells); cells treated with 0.005 mg/mL of peptide SEQ ID NO: 4-NH₂; cells treated with 0.01 mg/mL of peptide SEQ ID NO: 4-NH₂; and cells treated with 0.015 mg/mL of peptide SEQ ID NO: 4-NH₂.
Figure 9 shows the results obtained for procollagen I alpha 1 release in example 8 for the treatment of HDFa cells for 48 h with the peptide SEQ ID NO: 4-NH₂ at 0.01, 0.015 or 0.05 mg/mL. Procollagen I alpha 1 release is expressed as a percentage with regard to the basal condition (untreated cells) which is established as 100%. The y-axis shows the percentage of procollagen I alpha 1 release with regard to the basal condition. The x-axis shows the different treatment groups, being the bars from left to right: basal condition (untreated cells); cells treated with 0.01 mg/mL of peptide SEQ ID NO: 4-NH₂; cells treated with 0.015 mg/mL of peptide SEQ ID NO: 4-NH₂; and cells treated with 0.05 mg/mL of peptide SEQ ID NO: 4-NH₂.

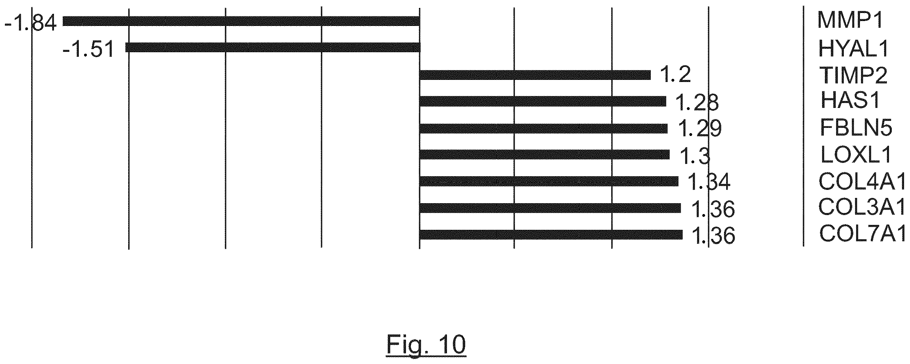

Figure 10 shows the gene expression results obtained in example 10 for the treatment of HDFa cells with peptide SEQ ID NO: 4-NH₂ at 24 h with 0.01 mg/mL of the peptide (MMP1, COL3A1 and COL4A1), at 24 h with 0.05 mg/mL of the peptide (TIMP2, FBLN5, LOXL1 and COL7A1), at 6 h with 0.005 mg/mL of the peptide (HYAL1); and at 6 h with 0.05 mg/mL of the peptide (HAS1). Each of the bars refers to one gene, from top to bottom: MMP1, HYAL1, TIMP2, HAS1, FBLN5, LOXL1, COL4A1, COL3A1 and COL7A1. Bars growing towards the left (this is, with a negative value) show a downregulation of the gene. Bars growing towards the right (this is, with a positive value) show an upregulation of the gene.

### EXAMPLES

Abbreviations:
The abbreviations used for amino acids follow the 1983 IUPAC-IUB Joint Commission on Biochemical Nomenclature recommendations outlined in Eur. J. Biochem. (1984) 138:937.

Ala, alanine; Arg, arginine; Asn, Asparagine; Asp, aspartic acid; Boc, tert-butyloxycarbonyl; cDNA, Complementary DNA; C-terminal, carboxy-terminal; Cys, cysteine; DCM, dichloromethane; DIPCDI, N,N'-diisopropylcarbodiimide; DMF, N,N-dimethylformamide; equiv, equivalent; ESI-MS, electrospray ionization mass spectrometry; Fmoc, 9-fluorenylmethyloxycarbonyl; Glu, Glutamic acid; Gln, Glutamine; HBSS, Hanks' Balanced Salt Solution; HEPES, 4-(2-hydroxyethyl)-1-piperazineethanesulfonic acid; His, histidine; HOBt, 1-hydroxybenzotriazole; HPLC, high performance liquid chromatography; HRP, Horseradish peroxidase; Ile, Isoleucine; INCI, International Nomenclature of Cosmetic Ingredients; LSGS, Low Serum Growth Supplement; Lys, Lysine; MBHA, p-methylbenzhydrylamine; Me, methyl; MeCN, acetonitrile; MEM, Minimum Essential Medium; MeOH, methanol; Met, Methionine; N-terminal, amino-terminal; Pbf, 2,2,4,6,7-pentamethyldihydrobenzofuran-5-sulfonyl; Phe, phenylalanine; Pro, proline; RNA, Ribonucleic acid; RT, room temperature; RT-qPCR, Quantitative reverse transcription Polymerase Chain Reaction; Ser, serine; SKM-D, Skeletal Muscle Cell Differentiation Medium; SKM-M, Skeletal Muscle Cell Growth Medium; tBu, *tert*-butyl; TFA, trifluoroacetic acid; TIS, triisopropylsilane; TPA, 12-O-tetradecanoylphorbol-13-acetate; Trp, Tryptophan; Trt, triphenylmethyl or trityl; Tyr, tyrosine; Val, valine.

Regarding the chemical synthesis procedures included in the examples, it is noted that all synthetic processes were carried out in polypropylene syringes fitted with porous polyethylene discs or Pyrex^{®} reactors fitted with porous plates. All the reagents and solvents were synthesis quality and were used without any additional treatment. The solvents and soluble reagents were removed by suction. The Fmoc group was removed with piperidine-DMF (2:8, volume/volume, v/v) (at least 1x1 min, 2x10 min, 5 mL/g resin) (Lloyd Williams P. et al., Chemical Approaches to the Synthesis of Peptides and Proteins, CRC, 1997, Boca Raton (Fla., USA)). Washes between stages of deprotection, coupling, and, again, deprotection, were carried out with DMF (3x1 min) and DCM (3x1 min) each time using 10 ml solvent/g resin. Coupling reactions were performed with 3 ml solvent/g resin. The control of the couplings was performed by carrying out the ninhydrin test (Kaiser E. et al., Anal. Biochem., 1970, 34: 595598). All synthetic reactions and washes were carried out at RT.

### Example 1. Synthesis and preparation of the peptides.

### a) Obtaining Fmoc-AA1-AA2-AA3-AA4-AA5-AA6-Rink-MBHA-resin, wherein AA1 is L-Lys; AA2 is L-Lys; AA3 is L-His: AA4 is L-Phe: AA5 is L-Trp; and AA6 is L-Ala.

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 2.33 g of Fmoc-L-Ala-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 3.94 g of Fmoc-L-Trp(Boc)-OH (7.5 mmol; 3 equiv); subsequently 2.90 g of Fmoc-L-Phe-OH (7.5 mmol; 3 equiv); subsequently 4.64 g of Fmoc-L-His(Trt)-OH (7.5 mmol; 3 equiv); subsequently 3.51 g Fmoc-L-Lys(Boc)-OH (7.5 mmol; 3 equiv) and subsequently 3.51 g of Fmoc-L-Lys(Boc)-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

### b) Obtaining Fmoc-AA1-AA2-AA3-AA4-AA5-Rink-MBHA-resin, wherein AA1 is L-Lys; AA2 is L-Asp; AA3 is L-Arg; AA4 is L-Val and AA5 is L-Tvr.

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 3.44 g of Fmoc-L-Tyr(tBu)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 2.55 g of Fmoc-L-Val-OH (7.5 mmol; 3 equiv); subsequently 4.86 g of Fmoc-L-Arg(Pbf)-OH (7.5 mmol; 3 equiv); subsequently 3.08 g of Fmoc-L-Asp(tBu)-OH (7.5 mmol; 3 equiv) and subsequently 3.51 g of Fmoc-L-Lys(Boc)-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

Using the synthesis procedures mentioned above, with the required selection of amino acids, the following sequences were synthesized:
Arg-Trp-Gln-Lys-Asn-Gln-NH₂ (SEQ ID NO: 1-NH₂);
Lys-Arg-Arg-Tyr-Tyr-Cys-NH₂ (SEQ ID NO: 2-NH₂);
Lys-His-Arg-Ser-Tyr-NH₂ (SEQ ID NO: 3-NH₂);
Lys-Asp-Arg-Val-Tyr-NH₂ (SEQ ID NO: 4-NH₂);
Ile-Glu-Pro-Tyr-Tyr-Val-NH₂ (SEQ ID NO: 5-NH₂); and
Lys-Lys-His-Phe-Trp-Ala-NH₂ (SEQ ID NO: 6-NH₂)

### c) Removal of Fmoc N-Terminal protective group of the peptides synthesized in accordance with a) or b).

The N-terminal Fmoc group of the peptidyl resins obtained in a) or b) above, was deprotected with 20% (volume/volume, hereinafterv/v) piperidine in DMF (1×1 min+2×10 min) (Lloyd Williams P. et al. (1997) Chemical Approaches to the Synthesis of Peptides and Proteins. CRC, Boca Raton (Fla., USA)). The peptidyl resins were washed with DMF (5×1 min), DCM (4×1 min), and dried under vacuum.

### d) Cleavage process from the polymeric support of the peptidyl resins obtained in accordance with c).

Weights were normalized. 200 mg of the dried peptidyl resin obtained in c) were treated with 5 mL of TFA/TIS/H₂O (90:5:5, in volume) for 2 hours at room temperature under stirring. The filtrates were collected and precipitated using 50 mL (8 to 10-fold) of cold diethyl ether. The ethereal solutions were evaporated to dryness at reduced pressure and room temperature, the precipitates were redissolved in 50% (v/v) MeCN in H₂O and lyophilized.

### e) Characterization of the peptides synthesized and prepared in accordance with d).

HPLC analysis of the peptides obtained in accordance with d) was carried out with a Shimadzu equipment (Kyoto, Japan) using a reverse-phase column (150x4.6 mm, XBridge Peptide BEH C18, 3.5 µm, Waters, USA) in gradients of MeCN (+0.036% (v/v) TFA) in H₂O (+0.045% (v/v) TFA) at a flow rate of 1.25 mL/min and detection was carried out at 220 nm. All peptides showed a purity exceeding 80%. The identity of the peptides obtained was confirmed by ESI-MS in a Water ZQ 4000 detector using MeOH as the mobile phase and a flow rate of 0.2 mL/min. Results obtained demonstrated that peptides SEQ ID NO: 1- NH₂, SEQ ID NO: 2- NH₂, SEQ ID NO: 3- NH₂, SEQ ID NO: 4- NH₂, SEQ ID NO: 5- NH₂ and SEQ ID NO: 6- NH₂ were correctly and effectively synthesized.

### Example 2. Analysis of the toxicity of the peptides of the present invention

In this case the MTT (3-[4,5-dimethylthiazol-2-yl]-2,5- diphenyltetrazolium bromide) assay was used to evaluate the cytotoxic potential of the peptides of the present invention on HEKa (Human Epidermal Keratinocytes from adult skin) and HDFa (Human Dermal Fibroblasts from adult skin) primary cells.

For this example, peptide SEQ ID NO: 4-NH₂ was used and it was prepared in accordance with example 1.

A stock solution of the peptide was prepared in distilled water at 25 mg/mL. This stock solution was further diluted to obtain the final working concentrations (0.001, 0.005, 0.01, 0.05, 0.1, 0.5 and 1 mg/mL) in the corresponding culture medium.

Untreated cells were used as negative control samples (Basal).

Briefly, HEKa and HDFa cells were seeded in 96-well plates at a concentration of 1×10⁵ cells/mL in a final volume of 200 µL of medium (20,000 cells/well). HEKa cells were incubated in Dermal Cell Basal Medium supplemented with the Keratinocytes Growth Kit; and HDFa cells were cultured in Medium 106 supplemented with 2% of LSGS. Cells were incubated at 37°C, 5% CO₂ and saturated humidity.

After 24 h of incubation, cells were treated with different concentrations of the peptide (0.001, 0.005, 0.01, 0.05, 0.1, 0.5 and 1 mg/mL) in triplicates, for MTT evaluation. After 24 h of treatment with the peptide, medium was completely removed, and 100 µL of 5 mg/mL MTT solution in supplemented medium were added to each well and cells were incubated at 37°C for 3 h. After incubating with MTT, the medium of each well was aspirated and 150 µL of DMSO were added in order to solubilise the formazan crystals formed. The plates were placed on a shaker for 5 minutes for complete solubilisation of the crystals and then the absorbance at 545 nm of each well was determined, using the microplate reader Multiskan FC, which is directly proportional to the number of living cells in culture.

Absorbance values were normalized using the data obtained for non-treated cells (control), obtaining the % of viability shown in Figures 1A and 1B. Each condition was performed in triplicate (n=3) in 3 independent experiments (N=3).

Concentrations of peptide for which viability is above 80% are considered safe for efficacy testing. On the contrary, concentrations for which viability goes below 80% are considered potentially toxic and are not used for efficacy testing purposes.

Obtained results appear summarized in Figures 1A (viability in HEKa cells) and 1B (viability in HDFa cells). As can be directly derived from said figures, viability in HEKa cells, after treatment with 0.001 to 0.5 mg/mL of the tested peptide, is above 80%; and viability in HDFa cells after treatment with 0.001 to 1 mg/mL of the tested peptide, is above 80%, which demonstrates the safety of the peptides of the present invention.

### Example 3. Analysis of the modulation of sodium channel activity or signalling

For this example, peptides SEQ ID NO: 1-NH₂, SEQ ID NO: 2-NH₂ and SEQ ID NO: 3-NH₂ were prepared in accordance with example 1.

Using human NaV1.3 stably transfected into Chinese Hamster Ovary (CHO) cells, electrophysiology was performed to measure ion channel activity using the QPatch48 HT (Sophion, Denmark). Experiments were performed in triplicate and using a concentration of peptide of 10 µM. A separate negative control was performed for each of the peptides. As positive control Amitriptyline was used.

The results obtained in this example appear summarized in Figure 2. As can be derived from said figure, all three peptides displayed inhibition of NaV1.3 inward currents and thus they had a less negative current (Amps). Therefore, all three peptides were able to block or modulate the sodium channel Nav1.3 (decrease activity or signal). In addition, surprisingly, SEQ ID NO: 2-NH₂ and SEQ ID NO: 3-NH₂ were able to perform an increased and statistically significant effect (p=0.018 and p=0.039, respectively).

### Example 4. Analysis of the modulation of sodium channel activity or signalling

For this example, peptides SEQ ID NO: 2-NH₂, SEQ ID NO: 3-NH₂, SEQ ID NO: 4-NH₂, SEQ ID NO: 5- NH₂ and SEQ ID NO: 6- NH₂ were prepared in accordance with example 1.

Using SHSY-5Y cells, electrophysiology was performed to measure ion channel activity using the QPatch48 HT (Sophion, Denmark). The samples tested were:
- Negative control: 0.05% (v/v) Bovine Serum Albumin
- Positive control: Tetradotoxin (TTX) at a concentration of 3 nM, 10 nM and 30 nM.
- SEQ ID NO: 2-NH₂ at a concentration of 0.005 mg/mL, 0.01 mg/mL and 0.05 mg/mL.
- SEQ ID NO: 3-NH₂ at a concentration of 0.005 mg/mL, 0.01 mg/mL and 0.05 mg/mL.
- SEQ ID NO: 4-NH₂ at a concentration of 0.005 mg/mL, 0.01 mg/mL and 0.05 mg/mL.
- SEQ ID NO: 5- NH₂ at a concentration of 0.005 mg/mL, 0.01 mg/mL and 0.05 mg/mL.
- SEQ ID NO: 6- NH₂ at a concentration of 0.005 mg/mL, 0.01 mg/mL and 0.05 mg/mL.

The percentage inhibition for each test dose application period was calculated as a reduction in mean cursor value (peak current) relative to the cursor value measured at the end of the vehicle control (pre-drug) and normalised to the maximum inhibition recorded with the supramaximal addition of TTX.

The obtained results appear summarized in Figure 3 which shows the percentage of inhibition (or blockage) of sodium channels (in the inactivated state). It can be seen that all the tested peptides effectively modulated the sodium channels (decrease in activity or signal) in a dose dependent manner. The peptide that obtained the best results at the two higher concentrations tested was SEQ ID NO: 4-NH₂. On its side, the peptide which performed the best at the lower concentration tested was SEQ ID NO: 5-NH₂.

The results showed that peptides of the present invention showed modulatory effects similar to that achieved with low concentrations of TTX (higher concentrations of TTX yielded full blocking effect).

### Example 5. Analysis of noradrenaline levels

For this example, peptides SEQ ID NO: 4-NH₂ and SEQ ID NO: 6- NH₂ were prepared in accordance with example 1.

In this case, noradrenaline (NA) levels were measured in differentiated SHSY5Y cells (human neuronal model). This test was used to measure if the candidate was able to modulate neurotransmitter release.

Human SH-SY5Y cells were seeded in triplicates in 24-well plates at a cell density of 250,000 cells/well. SH-SY5Y differentiated towards a neuronal phenotype with retinoic acid for 1 week and then treated with 0.05 and 0.1 mg/mL of the peptides mentioned above for 30 minutes.

After that, cells were washed and treated with 100 nM 12-0-tetradecanoyl-phorbol-13-acetate (TPA) first for 8 minutes, for release enhancement, and then with 100 mM KCI for 5 extra minutes in order to achieve an evoked release of neurotransmitters, all in HBSS with 20 mM HEPES buffer. Finally, cells were lysed and homogenized directly on wells through 10 passes through 0.8 mm gauge needle. Homogenized cell suspension was centrifuged at maximum speed for 15 minutes at 4°C. Supernatant was collected and kept at -80°C until total noradrenaline was quantified through enzyme-linked immunosorbent assay (ELISA), with a human Noradrenaline ELISA kit following manufacturer's instructions. Total protein content of each sample was quantified through Micro BCA Protein Assay kit in order to normalize data.

Each condition was performed at least in triplicate (n=3) in three independent experiments (N=3) and was normalized with regard to the total protein of the sample and with regard to the noradrenaline obtained for the positive control (sample without peptide but with stimulation) to obtain the percentage with regard to the positive control.

The results obtained appear summarized in Figures 4 and 5. As it can be directly derived from said figures, the peptides tested (as a representation or example of the peptides of the present invention) showed lower levels of noradrenaline (a reduction of more than 30%) and, hence, provided a botox-like effect. Thus, the treatment showed to modulate neurotransmitter release in stimulated and differentiated SH-SY5Y cells, which demonstrates that the peptides of the present invention provide for an anti-wrinkling activity.

### Example 6. Analysis of the expression muscle contraction-related genes

For this example, peptide SEQ ID NO: 4-NH₂ was prepared in accordance with example 1.

A stock solution of the peptide was prepared in milli-Q water at 25 mg/mL. This stock was further diluted in the corresponding supplemented medium to obtain the final working concentrations of 0.01 and 0.005 mg/mL. Untreated cells were used as negative control samples.

The relevant genes for which the expression was measured were: SCN1B (Sodium Voltage-Gated Channel Beta Subunit 1), SCN4A (Sodium Voltage-Gated Channel Alpha Subunit 4), SCN3A (Sodium Voltage-Gated Channel Alpha Subunit 3), CALM3 (Calmodulin 3) and RAPSN (Receptor Associated Protein of the Synapse). The primers used for each of the genes were commercially available TaqMan Gene Expression Assay probes. The experiment was performed in vitro in myocytes, two concentrations of the peptide were tested (0.01 mg/mL and 0.005 mg/mL) at 24h.

Briefly, human skeletal muscle myoblasts were seeded in 12-well plates at a density of 1 × 10⁵ cells/well and maintained at standard culture conditions (SKM-M medium supplemented with 1 % Antibiotic/Antimycotic; 37°C, 95% Relative Humidity, 5% CO₂) for 48-72 hours until the plated myoblasts reached 80-90% confluence. Then, growth medium was replaced by SKM-D medium in order to induce myotube differentiation (differentiation into myocytes) and fresh medium was added every 2-3 days. After 6-7 days from the initiation of the differentiation process, myocytes were treated with the above-mentioned peptide at 0.01 or 0.005 mg/mL for additional 24 hours. Untreated cells were used as basal control. Cells were finally lysed and RNA extraction was performed using Qiagen RNeasy Mini kit following manufacturer's instructions. Purified RNAs were used to generate the corresponding cDNAs by reverse transcription which served as templates for amplification. RT-qPCR was performed with the panel of TaqMan assay probes specified above and 2x gene expression Master Mix using StepOne plus Real-Time PCR instrument. Amplification included 40 cycles of: 15 seconds at 95°C (denaturation) and 1 minute at 60°C (Annealing and extension).

The obtained data were analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT calibrator sample
3. Calculate the ΔΔCT: ΔΔCT = ΔCT test sample - ΔCT calibrator sample
4. Obtain ratio by 2^{-ΔΔCT}

The results obtained appear summarized in Figures 6 and 7, which show that the peptide SEQ ID NO: 4-NH₂ (as an example of the peptides of the present invention) was able to modulate the expression in myocytes of the relevant genes tested and, therefore, the peptide is able to modulate (downregulate) the expression of sodium channels and muscle contraction-related genes in muscle cells.

### Example 7. Analysis of the synthesis of elastin

For this example, peptide SEQ ID NO: 4-NH₂ was prepared in accordance with example 1.

A stock solution of the peptide was prepared in water at 25 mg/mL. This stock was further diluted to obtain the final working concentrations of 0.005, 0.01 or 0.015 mg/mL in complete medium (Medium 106 supplemented with 2% LSGS). Untreated cells were used as basal condition.

For this study, HDFa cells were seeded in a 12-well plate at a density of 120,000 cells/well and maintained at standard culture conditions (Medium 106 + 2% LSGS; 37°C, 95% Relative Humidity, 5% CO₂) for 24 hours. Then, fibroblasts were treated for 48 hours with 0.005, 0.01 or 0.015 mg/mL of the peptide mentioned above. After treatment, cells were harvested for elastin quantification with the Fastin Elastin Assay Kit, according to manufacturer instructions. Each condition was performed at least in triplicate (n=3) in three independent experiments (N=3).

Obtained results appear summarized in Figure 8. HDFa treated with 0.01 and 0.015 mg/mL of the peptide of the present invention showed an increase of elastin levels, when compared with untreated samples. These results suggest a potential role of the peptides of the present invention increasing skin elasticity as well as improving skin structural integrity and improving (prevent, treat or reduce) looser and/or sagging skin. In addition, the increase in elastin synthesis or production also demonstrates that the peptides of the present invention are able to improve skin firmness, and wound-healing capabilities.

### Example 8. Analysis of procollagen I alpha 1 release

For this example, peptide SEQ ID NO: 4-NH₂ was prepared in accordance with example 1.

A stock solution of the peptide was prepared in water at 25 mg/mL. This stock was further diluted to obtain the final working concentrations of 0.01, 0.015 or 0.05 mg/mL in complete medium (Medium 106 supplemented with 2% LSGS). Untreated cells were used as basal condition.

For this study, HDFa cells were seeded in a 24-well plate at a density of 100,000 cells/well and maintained at standard culture conditions (Medium 106 supplemented with 2% LSGS; 37°C, 95% Relative Humidity, 5% CO₂) for 24 hours.

After 24 hours, cells were treated for 48 h with the peptide of the present invention mentioned above (0.01, 0.015 and 0.05 mg/mL). After treatments, supernatants were collected and centrifuged in order to eliminate cell debris and were kept at -80°C until they were used for procollagen I alpha 1 quantification with a human procollagen I alpha 1 DuoSet ELISA kit according to manufacturer instructions. Each condition was performed at least in triplicate (n=3) in three independent experiments (N=3).

Obtained results appear summarized in Figure 9. As can be directly derived from said figure, procollagen I alpha 1 protein release was increased at all tested concentrations, in a dose-dependent manner, reaching up to 53% significant increase at the highest tested concentration (0.05 mg/mL). These results demonstrate that the peptides of the present invention provide for Collagen type I release.

### Example 9. Analysis of the synthesis of hyaluronic acid.

For this example, peptide SEQ ID NO: 4-NH₂ was prepared in accordance with example 1.

In this case the objective of this experiment was to evaluate *in vitro* the capability of the peptides of the present invention to increase Hyaluronic Acid (HA) synthesis or production in human skin fibroblasts. This evaluation was carried out by determination of HA amount after cell treatment with the peptide (at the different concentrations noted below), for 48 hours.

### Briefly:

The experimental groups were:
- Untreated cell cultures (negative control).
- Cells treated with the peptide of the present invention (at 0.005 mg/mL, 0.01 mg/mL or 0.05 mg/mL).

Treatment was performed for 48 hours. Treatments were performed in triplicate and in two different experimental sessions.

The experimental model used in this study were Human Fibroblast (SF-TY JCRB) cultivated in MEM with fetal bovine serum 10% and 1% antibiotics at 37°C, 5% CO₂.

Cells were seeded in 96-well plate at 1 × 10⁴ cells/well and maintained for 24h at standard culture conditions (37°C, 95% Relative Humidity, 5% CO₂) before the treatment was applied.

At the end of the experimental period, the determination of Hyaluronic synthesis was carried out by means of ELISA method. Commercial kits were used for this purpose. The test kit employed a two-site sandwich ELISA to quantitate HA in samples. Briefly, an antibody specific for HA was pre-coated onto a microplate; Standards and samples were pipetted into the wells and any HA present got bound by the immobilized antibody; after removing any unbound substances, a biotin-conjugated antibody specific for HA was added to the wells; after washing, Streptavidin conjugated Horseradish Peroxidase (HRP) was added to the wells; following a wash to remove any unbound avidin-enzyme reagent, a substrate solution was added to the wells and colour developed in proportion to the amount of HA bound in the initial step. The colour development was stopped and the intensity of the colour was measured. A calibration curve of known concentrations of standard hyaluronic acid was used to perform the determination of the tested samples.

Obtained results appear summarized in Table 1. The results are expressed as % of HA content in cell culture medium and cell lysate, which is calculated by stablishing the content of HA in the negative control as 100% and making the appropriate comparison with the rest of the samples. The % variation in HA content between negative controls and samples shows the efficacy of the tested peptide of the present invention to increase Hyaluronic Acid synthesis or production.

**Table 1. Results obtained for HA content in the first experimental session at 48 hours. ** is statistically significant (p<0.01); *** is statistically significant (p<0.001).**

| Sample | % HA content | % variation with regard to negative control |
|---|---|---|
| Negative control (untreated cells) | 100 | - |
| Cells treated with SEQ ID NO: 4-NH₂ at 0.005 mg/mL | 107.57** | 7.57** |
| Cells treated with SEQ ID NO: 4-NH₂ at, 0.01 mg/mL | 121.75*** | 21.75*** |
| Cells treated with SEQ ID NO: 4-NH₂ at 0.05 mg/mL | 139.21*** | 39.21*** |

As it can be readily derived from Table 1, the peptides of the present invention (as exemplified by SEQ ID NO: 4-NH₂) provide an increase in the synthesis or production of HA.

### Example 10. Analysis of the expression of ECM-related genes

For this example, peptide SEQ ID NO: 4-NH₂ was prepared in accordance with example 1.

A stock solution of the peptide was prepared in milli-Q water at 25 mg/mL. This stock was further diluted in the corresponding supplemented medium to obtain the final working concentrations of 0.05, 0.01 and 0.005 mg/mL. Untreated cells were used as negative control samples.

The relevant genes for which the expression was measured were: MMP1 (matrix metallopeptidase 1), HYAL1 (hyaluronidase 1), TIMP2 (TIMP metallopeptidase inhibitor 2), HAS1 (hyaluronan synthase 1), FBLN5 (fibulin 5), LOXL1 (lysyl oxidase like 1), COL4A1 (collagen type IV alpha 1 chain), COL3A1 (collagen type III alpha 1 chain) and COL7A1 (collagen type VII alpha 1 chain). The primers used for each of the genes were commercially available TaqMan Gene Expression Assay probes.

Briefly, HDFa cells were seeded in 12-well plates at a density of 1.5 × 10⁵ cells/well and maintained at standard culture conditions (106 Medium supplemented with 1% LSGS; 37°C, 95% Relative Humidity, 5% CO₂) for 24 hours. Then, cells were treated with the above-mentioned peptide at 0.005, 0.01 or 0.05 mg/mL for additional 6 or 24 hours.

Cells were finally lysed and RNA extraction was performed using Qiagen RNeasy Mini kit following manufacturer's instructions. Purified RNAs were used to generate the corresponding cDNAs by reverse transcription which served as templates for amplification. RT-qPCR was performed with the panel of TaqMan assay probes specified above and 2x gene expression Master Mix using StepOne plus Real-Time PCR instrument. Amplification included 40 cycles of: 15 seconds at 95°C (denaturation) and 1 minute at 60°C (Annealing and extension).

The obtained data were analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT calibrator sample
3. Calculate the ΔΔCT: ΔΔCT = ΔCT test sample - ΔCT calibrator sample
4. Obtain ratio by 2^{-ΔΔCT}

The results obtained appear summarized in figure 10, which shows that the peptide SEQ ID NO: 4-NH₂ (as an example of the peptides of the present invention) was able to modulate the expression in fibroblasts of the relevant genes of the ECM tested inducing: at 24 h with 0.01 mg/m of the peptide, a downregulation of MMP1 and an upregulation of COL3A1 and COL4A1; at 24 h with 0.05 mg/mL of the peptide, an upregulation of TIMP2, FBLN5, LOXL1 and COL7A1; at 6 h with 0.005 mg/mL of the peptide, a downregulation of HYAL1; and at 6 h with 0.05 mg/mL of the peptide, an upregulation of HAS1. MMP1 and HYAL1 are genes related with the degradation of the ECM and HA, respectively. TIMP2, HAS1, FBLN5, LOXL1, COL4A1, COL3A1 and COL7A1 are genes related with the synthesis and effective maintenance of the ECM. Therefore, the expression profile obtained with the peptides of the present invention (as exemplified by SEQ ID NO: 4-NH₂) demonstrates their usefulness in the maintenance and synthesis or increase of the ECM.

Therefore, as it has been demonstrated in the examples included above, the peptides of the present invention were effectively produced and they showed the activities mentioned throughout the specification:
- Modulation or decrease of the activity or signalling of voltage-gated sodium channels.
- Modulation or decrease of the expression of voltage-gated sodium channels.
- Decrease in the levels of noradrenaline (and, hence, inhibition of the release of noradrenaline).
- Increase in the production of elastin.
- Increase in the release of procollagen I alpha 1.
- Increase in the levels of hyaluronic acid.
- Favourable regulation of ECM related genes.

## Claims

1. Peptide of between 4 and 8 amino acids capable of decreasing the activity and/or signal of at least one type of sodium channel; its acceptable isomers, salts, solvates, derivatives and/or mixtures thereof.

2. Peptide in accordance with claim 1, **characterized in that** the peptide is capable of downregulating the expression of at least one type of sodium channel and/or it is capable of decreasing the levels of at least one neurotransmitter and/or it is capable of maintaining and/or synthesizing ECM.

3. Peptide in accordance with claim 1 or claim 2, **characterized in that** the amino acid of the first position and/or of the second position is a charged amino acid (positively or negatively).

4. Peptide in accordance with any one of claims 1 to 3, **characterized in that** the first three amino acids are charged amino acids (positively or negatively).

5. Peptide in accordance with claim 4, **characterized in that** the amino acids in the first and in the third position are positively charged amino acids and the amino acid in the second position is a positively or negatively charged amino acid.

6. Peptide in accordance with any one of claims 3 to 5, **characterized in that** the rest of the amino acids are polar without charge, apolar and/or aromatic.

7. Peptide in accordance with any one of claims 3 to 6, **characterized in that** the charged amino acid in position 1 is Lys.

8. Peptide in accordance with any one of claims 3 to 7, **characterized in that** the first three amino acids of the peptide are: Lys-Arg-Arg, Lys-His-Arg, Lys-Asp-Arg or Lys-Lys-His.

9. Peptide in accordance with any of claims 1 to 8, **characterized in that** it has the sequence of formula (I):
R₁-AA1-AA2-AA3-AA4-AA5-AA6-R₂
(R₁-SEQ ID NO: 7-R₂)
wherein
AA1 is selected from Lys, Arg or His;
AA2 is selected from Lys, Arg, His, Asp or Glu;
AA3 is selected from Lys, Arg or His;
AA4 is selected from Gly, Ala, Val, Leu, Met, Ile, Ser, Thr, Cys, Pro, Asn, Gln, Phe, Tyr or Trp;
AA5 is selected from Phe, Tyr or Trp;
AA6 is absent or is selected from Gly, Ala, Val, Leu, Met, Ile, Ser, Thr, Cys, Pro, Asn or Gln;
R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, R₁ and R₂ are absent and AA1 is directly bound to the last amino acid of the peptide.

10. Peptide in accordance with any one of claims 1 to 3, **characterized in that** the peptide is selected from:
- R₁-Arg-Trp-Gln-Lys-Asn-Gln-R₂;
- R₁-Lys-Arg-Arg-Tyr-Tyr-Cys-R₂; or
- R₁-Lys-His-Arg-Ser-Tyr-R₂
- R₁-Lys-Asp-Arg-Val-Tyr-R₂;
- R₁-Ile-Glu-Pro-Tyr-Tyr-Val-R₂; or
- R₁-Lys-Lys-His-Phe-Trp-Ala-R₂
and wherein:
R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl;
alternatively, R₁ and R₂ are absent and AA1 is directly bound to the last amino acid of the peptide.

11. Peptide in accordance with any one of claims 9 or 10, **characterized in that** R₁ is H and/or R₂ is NH₂.

12. Composition comprising a peptide in accordance with any one of claims 1 to 11.

13. Use as a cosmetic of a peptide in accordance with any one of claims 1 to 11 or of a composition in accordance with claim 12 in a subject in need thereof.

14. Use as a cosmetic in accordance with claim 13, **characterized in that** the use as a cosmetic is to reduce, prevent and/or eliminate signs of skin aging.

15. Use as a cosmetic in accordance with claim 14, **characterized in that** the signs of skin aging are wrinkles.
